# EUROPEAN PATENT APPLICATION

(11) **EP 3 932 451 A1**
(43) Date of publication of application: **05.01.2022**
(21) Application number: 20911310.9
(22) Date of filing: 15.10.2020
(51) Int. Cl.: A61M 5/165, B01D 35/02, B01D 35/30, A61M 5/168

(54) **FILTER SHEET STACKING STRUCTURE OF FILTER FOR INTRAVENOUS INFUSION**

(30) Priority: 12.03.2020 KR 20200030903; 06.04.2020 KR 20200041785
(71) Applicant: MEDI LINE ACTIVE KOREA CO., LTD., Gyeonggi-do 15455 (KR)
(72) Inventor: KANG, Dae Won, Guro-gu, Seoul 08230 (KR); LEE, Young Kyu, Gangnam-gu Seoul 06175 (KR)
(74) Representative: Caspary, Karsten
(86) International application number: PCT/KR2020/014079
(87) International publication number: WO 2021/182703

(57) **Abstract**

The present invention relates to a filter laminating structure for an intravenous infusion filter that is capable of allowing a filter body to be supported against a filter type of support body, thereby preventing the filter body from being broken or damaged even by the injection of medicine under high pressure, and the filter laminating structure including an upper filter housing having a medicine injection hole adapted to inject medicine therethrough, a lower filter housing coupled to the underside of the upper filter housing, a filter body located at an upstream side in the flow of the medicine injected into the medicine injection hole to perform a filtering function for the medicine, and a support body located at a downstream side in the flow of the medicine on the underside of the filter body to support the filter body thereagainst.

## Description

### Cross Reference to Related Application of the Invention

The present application claims the benefit of Korean Patent Application No.10-2020-0030903 filed in the Korean Intellectual Property Office on March 12, 2020 and Korean Patent Application No.10-2020-0041785 filed in the Korean Intellectual Property Office on April 6, 2020, the entire contents of which are incorporated herein by reference.

### Field of the Invention

The present invention relates to a filter laminating structure for an intravenous infusion filter, and more particularly, to a filter laminating structure for an intravenous infusion filter that is capable of allowing a filter body performing a filtering function to be supported against a filter type of support body, thereby preventing the filter body from being broken or damaged even by the injection of medicine under high pressure.

### Background of the Related Art

When medicine for treatment in hospitals is injected into a patient's body, generally, the medicine is injected into the vein or subcutaneous fat. For the injection into the subcutaneous fat, a syringe is generally used, and for the injection into the vein at a given speed, a medical infusion line is generally used.

In this case, a filter is used to remove the foreign matters that may enter the medicine, and a membrane filter is commonly used as the filter.

A blood vessel contrast medium is used to obtain computed tomography (CT) or magnetic resonance image (MRI), but in this case, the medicine is injected at a maximum speed of 3 to 5 ml/sec. If only the membrane filter is used, a pressure is accumulated on the membrane filter, and when the membrane filter reaches the threshold point of breakage, it becomes broken or damaged, thereby losing its original function.

As the blood vessel contrast medium has a high viscosity, further, it has a remarkably higher pressure than general saline solution and distilled water.

So as to inject the blood vessel contrast medium into the vein, a media injector is used, but due to the high pressure applied from the media injector, the membrane filter may be easily broken or damaged. In this case, if filter powder having sizes of about 5 microns or more is generated from the broken or damaged membrane filter, the filter powder blocks the blood vessel to cause serious medical accidents.

So as to prevent the membrane filter from being broken or damaged in conventional practices, a mesh filter is located on the inlet side of a filter housing into which medicine is injected and a membrane filter is located on the outlet side of the filter housing.

Such a conventional filter structure has no support body for supporting the membrane filter so that the membrane filter may be broken or damaged in the same manner as the single filter structure.

### SUMMARY OF THE INVENTION

Accordingly, the present invention has been made in view of the above-mentioned problems occurring in the related art, and it is an object of the present invention to provide a filter laminating structure for an intravenous infusion filter that is provided with a membrane filter located on the inlet side into which medicine is injected to thus perform a filtering function for the medicine and a support body formed of a mesh or sintered filter and located on the underside of the membrane filter on the outlet side from which the medicine is discharged to thus support the membrane filter thereagainst, thereby maximizing the filtering efficiency, preventing the membrane filter from being broken or damaged due to high pressure injection, and optimizing the effectiveness and rigidity of a product.

To accomplish the above-mentioned object, according to the present invention, there is provided a filter laminating structure for an intravenous infusion filter, including: an upper filter housing having a medicine injection hole adapted to inject medicine therethrough; a lower filter housing coupled to the underside of the upper filter housing and having a medicine outlet adapted to discharge the medicine therethrough; a filter body located at an upstream side in the flow of the medicine injected into the medicine injection hole in the interiors of the upper filter housing and the lower filter housing to perform a filtering function for the medicine; and a support body located at a downstream side in the flow of the medicine on the underside of the filter body to support the filter body thereagainst.

According to the present invention, desirably, the filter body comes into close contact with top of the support body with respect to the upper filter housing into which the medicine is injected and is formed of a membrane filter having a high filtering efficiency.

According to the present invention, desirably, the support body is formed of a woven mesh filter or a sintered filter made by sintering under high temperature and high pressure compression and serves as a support for supporting the filter body thereagainst to prevent the filter body from being broken or damaged by the medicine injected at a high speed and high pressure.

According to the present invention, desirably, the support body comes into close contact with underside of the filter body with respect to the upper filter housing into which the medicine is injected to support the filter body thereagainst.

According to the present invention, desirably, the filter laminating structure further includes a check valve housing connected to the underside of the lower filter housing and having a check valve disposed therein.

### BRIEF DESCRIPTION OF THE DRAWINGS

The above and other objects, features and advantages of the present invention will be apparent from the following detailed description of the embodiments of the invention in conjunction with the accompanying drawings, in which:
FIG. 1 is an exploded perspective view showing a filter laminating structure for an intravenous infusion filter according to the present invention;
FIG.2 is a perspective view showing the filter laminating structure for an intravenous infusion filter according to the present invention;
FIG. 3 is a sectional view showing the internal configuration of the filter laminating structure for an intravenous infusion filter according to the present invention; and
FIG.4 is a sectional view showing the internal configuration in a coupled state of the filter laminating structure for an intravenous infusion filter according to the present invention.

### DETAILED DESCRIPTION OF THE EMBODIMENTS

The present invention may be modified in various ways and may have several exemplary embodiments. Specific exemplary embodiments of the present invention are illustrated in the drawings and described in detail in the detailed description. However, this does not limit the invention within specific embodiments and it should be understood that the invention covers all the modifications, equivalents, and replacements within the idea and technical scope of the invention.

In the description, the thicknesses of the lines or the sizes of the components shown in the drawing may be magnified for the clarity and convenience of the description. Further, the terms as will be discussed later are defined in accordance with the functions of the present invention, but may be varied under the intention or regulation of a user or operator. Therefore, they should be defined on the basis of the whole scope of the present invention.

A term 'and/or' includes a combination of a plurality of relevant and described items or any one of a plurality of related and described items. When it is said that one element is described as being "connected" or "coupled" to the other element, one element may be directly connected or coupled to the other element, but it should be understood that another element may be present between the two elements. An expression referencing a singular value additionally refers to a corresponding expression of the plural number, unless explicitly limited otherwise by the context. In this application, terms, such as "comprise", "include", or "have", are intended to designate those characteristics, numbers, steps, operations, elements, or parts which are described in the specification, or any combination of them that exist, and it should be understood that they do not preclude the possibility of the existence or possible addition of one or more additional characteristics, numbers, steps, operations, elements, or parts, or combinations thereof.

In the description, when it is said that one member, such as a layer (film), area, pattern or structure is located "on" or "under" another member, such as a substrate, layer (film), area, pad, or pattern, it means that one member may come into contact with another member as well as yet another member may exist between the two members. Standards of "on or under" positions for the respective layers are explained with reference to the attached drawings.

Hereinafter, an explanation on a filter laminating structure for an intravenous infusion filter according to the present invention will be in detail given with reference to the attached drawing.

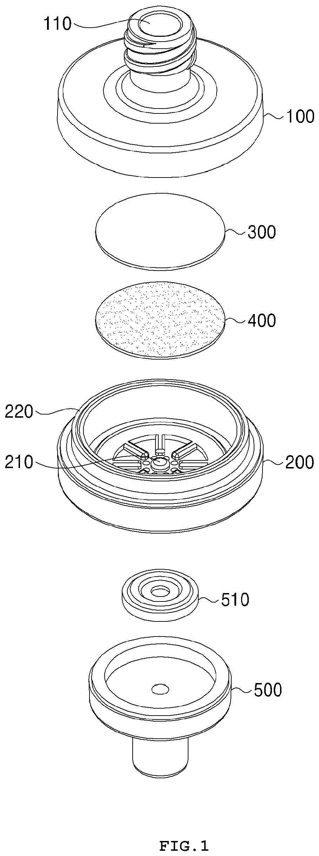

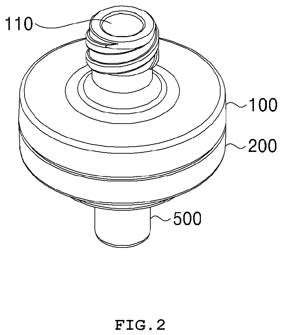

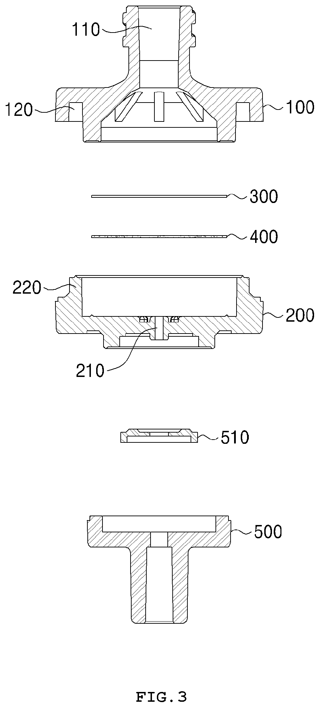

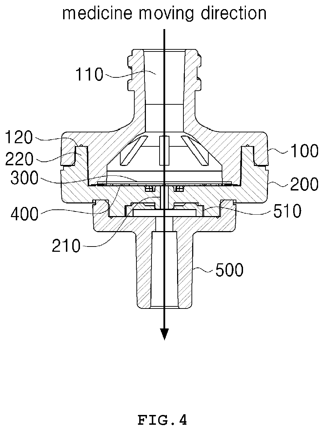

FIG. 1 is an exploded perspective view showing a filter laminating structure for an intravenous infusion filter according to the present invention, FIG.2 is a perspective view showing the filter laminating structure for an intravenous infusion filter according to the present invention, FIG.3 is a sectional view showing the internal configuration of the filter laminating structure for an intravenous infusion filter according to the present invention, and FIG.4 is a sectional view showing the internal configuration in a coupled state of the filter laminating structure for an intravenous infusion filter according to the present invention.

As shown in FIGS.1 to 4, a filter laminating structure for an intravenous infusion filter according to the present invention includes an upper filter housing 100, a lower filter housing 200, a filter body 300, a support body 400, and a check valve housing 500.

The upper filter housing 100 has a medicine injection hole 110 penetrated vertically thereinto to inject medicine therethrough.

Further, the upper filter housing 100 has a coupling groove 120 formed along the inner periphery of the underside thereof in such a manner as to be coupled to the lower filter housing 200.

The lower filter housing 200 is coupled to the underside of the upper filter housing 100 and has a medicine outlet 210 adapted to discharge the medicine passing through the upper filter housing 100 from the medicine injection hole 110 therethrough.

Further, the lower filter housing 200 has an insertion piece 220 protruding from top periphery thereof in such a manner as to be inserted into the coupling groove 120 to allow the upper filter housing 100 to be connected thereto.

After the insertion piece 220 is inserted into the coupling groove 120, further, it is melted in the coupling groove 120, so that the upper filter housing 100 and the lower filter housing 200 can be firmly fixed to each other.

The filter body 300 is located at an upstream side in the flow of the medicine injected into the medicine injection hole 110 in the interiors of the upper filter housing 100 and the lower filter housing 200 to perform a filtering function for the medicine.

Further, the filter body 300 comes into close contact with top of the support body 400 as will be discussed later with respect to the upper filter housing 100 into which the medicine is injected through the medicine injection hole 110.

Also, the filter body 300 is formed of a membrane filter to enhance a filtering efficiency for the medicine.

As the filter body 300 is formed of such a membrane filter, that is, the foreign matters, which may enter the medicine, can be optimizedly removed.

As shown in FIG.4, the filter body 300 is fittedly connected to the inner periphery of the underside of the upper filtering housing 100.

The support body 400 is located at a downstream side in the flow of the medicine toward the medicine outlet 210 on the underside of the filter body 300 between the upper filter housing 100 and the lower filter housing 200, thereby serving as a support for the filter body 300.

The support body 400 is formed of a woven mesh filter, and otherwise, the support body 400 is formed of a sintered filter made by sintering under high temperature and high pressure compression.

The mesh filter is made by weaving wires to the mesh form.

The sintered filter is made by bonding balls and pressurizing the balls under high pressure.

Further, the support body 400 serves to support the filter body 300 to prevent the filter body 300 from being broken or damaged by the medicine injected at a high speed and high pressure from the medicine injection hole 110.

That is, the support body 400 comes into close contact with underside of the filter body 300 with respect to the upper filter housing 100 into which the medicine is injected through the medicine injection hole 110 to thus support the filter body 300 thereagainst, so that the support body 400 serves as a support for the filter body 300 to prevent the filter body 300 as the membrane filter from being broken or damaged. Accordingly, a threshold point of the breakage of the membrane filter is raised up to a threshold point of the breakage of the mesh or sintered filter, so that the filter body 300 can be prevented from being broken or damaged.

The check valve housing 500 is coupled to the underside of the lower filter housing 200 and has a check valve 510 located therein to prevent the medicine from flowing back.

When the check valve housing 500 is coupled to the underside of the lower filter housing 200, further, it may be melted therein.

As the check valve housing 500 is coupled to the lower filter housing 200, the check valve 510 is located between the check valve housing 500 and the lower filter housing 200 to prevent the medicine passing through the upper filter housing 100, the filter body 300 and the support body 400 and next flowing to the lower filter housing 200 from flowing back.

Under the above-mentioned configuration, now, an operation of the filter laminating structure for an intravenous infusion filter according to the present invention will be explained.

The lower filter housing 200 with the medicine outlet 210 is coupled to the underside of the upper filter housing 100 with the medicine injection hole 110. In this case, in the interiors of the upper filter housing 100 and the lower filter housing 200, the filter body 300 as the membrane filter is provided, and further, the support body 400 as the mesh or sintered filter is provided on the underside of the filter body 300 with respect to the upper filter housing 100 into which the medicine is injected through the medicine injection hole 110.

Like this, the filter body 300 is located at the upstream side in the flow of the medicine with respect to the upper filter housing 100 to thus perform the filtering function, so that the filter body 300 as the membrane filter can remove the foreign matters entering the medicine.

Further, the support body 400 is located at the downstream side in the flow of the medicine on the underside of the filter body 300 with respect to the upper filter housing 100, so that the support body 400 as the mesh or sintered filter can support the filter body 300 as the membrane filter thereagainst to prevent the filter body 300 from being broken or damaged.

In addition, the filter body 300 and the support body 400 as the filters for filtering the medicine are doubly laminated on each other inside the upper filter housing 100 and the lower filter housing 200, thereby optimizing the filtering efficiency for the medicine.

Also, the check valve housing 500 with the check valve 510 is located on the underside of the lower filter housing 200, so that there is no need to additionally mount a check valve on an infusion line, thereby reducing a manufacturing cost.

As described above, the filter laminating structure for an intravenous infusion filter according to the present invention is configured to have the filter body as the membrane filter located at the upstream side in the flow of the medicine injected into the medicine injection hole in the interiors of the upper filter housing and the lower filter housing and to have the support body as the mesh or sintered filter located at the downstream side in the flow of the medicine on the underside of the filter body, thereby maximizing the filtering efficiency through the filter body and the support body and allowing the filter body as the membrane filter to be supported against the support body to prevent the filter body from being broken or damaged even by the medicine injected at a high speed and high pressure.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. A filter laminating structure for an intravenous infusion filter, comprising:
an upper filter housing having a medicine injection hole adapted to inject medicine therethrough;
a lower filter housing coupled to the underside of the upper filter housing and having a medicine outlet adapted to discharge the medicine therethrough;
a filter body located at an upstream side in the flow of the medicine injected into the medicine injection hole in the interiors of the upper filter housing and the lower filter housing to perform a filtering function for the medicine; and
a support body located at a downstream side in the flow of the medicine on the underside of the filter body to support the filter body thereagainst.

2. The filter laminating structure according to claim 1, wherein the filter body comes into close contact with top of the support body with respect to the upper filter housing into which the medicine is injected and is formed of a membrane filter having a high filtering efficiency.

3. The filter laminating structure according to claim 1, wherein the support body is formed of a woven mesh filter or a sintered filter made by sintering under high temperature and high pressure compression and serves as a support for supporting the filter body thereagainst to prevent the filter body from being broken or damaged by the medicine injected at a high speed and high pressure.

4. The filter laminating structure according to claim 1, wherein the support body comes into close contact with underside of the filter body with respect to the upper filter housing into which the medicine is injected to thus support the filter body thereagainst.

5. The filter laminating structure according to claim 1, further comprising a check valve housing connected to the underside of the lower filter housing and having a check valve disposed therein.
